# EUROPEAN PATENT SPECIFICATION

(11) **EP 2 411 402 B1**
(45) Date of publication and mention of the grant of the patent: **12.02.2014**
(21) Application number: 09786340.1
(22) Date of filing: 23.03.2009
(51) Int. Cl.: C07H 21/00, G01N 21/65

(54) **SYNTHESIS OF OLIGONUCLEOTIDE MEDIATED GOLD CORE- SILVER SHELL NANOPARTICLES**
SYNTHESE VON OLIGONUKLEOTIDHALTIGEN NANOPARTIKELN MIT EINEM GOLDKERN UND EINER SILBERBESCHICHTUNG
SYNTHESE DE NANOPARTICULES COMPRENANT UN NOYAU D'OR , UNE ENVELOPPE D'ARGENT ET DES OLIGONUCLEOTIDES

(43) Date of publication of application: 01.02.2012
(73) Proprietor: Yeditepe Universitesi, 34755 Istanbul (TR)
(72) Inventor: CULHA, Mustafa, 34755 Istanbul (TR)
(74) Representative: Dericioglu Kurt, Ekin
(86) International application number: PCT/IB2009/051197
(87) International publication number: WO 2010/109268

(56) References cited:
- FR-A1- 2 863 053
- US-A1- 2002 177 143
- US-A1- 2006 234 248

## Description

### Field of the Invention

The present invention is related to the synthesis of the oligonucleotide mediated gold core- silver shell nanoparticles that can be used in surface-enhanced Raman scattering (SERS), colorimetic based detections and imaging systems, and photothermal teraphy.

### Background Art

Surface enhanced Raman scattering (SERS) is a powerful spectroscopic technique to acquire molecular level information from the chemical and biological structures brought into close contact with noble metal surfaces such as gold and silver. The noble metal colloidal nanoparticles are commonly used in SERS applications [1-6]. In addition to their simple preparation and low cost, they can be now synthesized with different shape and size. With the recent advances in understanding of SERS mechanism and nanostructure preparation techniques, it is now possible to design nanostructured surfaces generating extraordinary enhancement and more reproducible results [7-9]. The necessity of generating overlapping surface plasmons for exceptionally high enhancement was one of the most important concepts for the further advancement of the technique [10,11].

The preparation of the core-shell nanoparticles was previously reported and the most important aspect of the preparation of such structures is their optimal performance though the surface Plasmon excitation for sensing and imaging, and their biocompatibility (12-29). Therefore, a noble metal such as silver or gold layer shell is prepared on dielectric cores such as SiO₂ and TiO₂ or a gold or silver core is coated with a dielectric layer [13-16]. In the former case, the surface Plasmon exciation wavelength of the core-shell nanoparticles depends on the shell thickness and it can be shifted into NIR region of the spectrum, which is more proper for biological applications such as cellular and biomdecical imaging and photothermal therapy. [13, 17,18]. In the latter case, the biocompatibility is maintained while keeping the sensitivity optimal. The gold nanoparticles coated with Raman reporter doped glass to use in multiplexing bioassays were demonstrated [19].

Another study demonstrated the applicability of the core-shell structures in cancer detection [20]. In that study, the silver nanoparticles were first embedded onto silica nanoparticles, and then a Raman reporter molecule was chemically bound to the Ag nanoparticles. Finally, the target antibody specific for the cancer marker was bound to the thin silica layer, which was prepared on the top of Ag nanoparticles. The Au core- silica shell nanoparticles embedded with Raman reporters was used in multiplexed detections [21].
The deposition of a noble metal directly onto another is another approach for the preparation of core-shell nanoparticles. This preparation relies on the reduction of gold or silver ions from their salts onto their metal nanoparticles [22-24]. The prepared core-shell nanoparticles (Ag core-Au shell and Au core-Ag shell) were tested for their SERS sperformance for possible imaging and detection and they showed significant improvement in SERS activity [22,23] The previously reported results later confirmed by another study and indicated that the Au-Ag core-shell nanoparticles were the best SERS active structures [24]. The use of the Ag core-Au shell nanoparticles for antigen detection based on SERS was reported after their simultaneous modification with a Raman reporter molecule and antibody [25]. In that study, the improvement in SERS enhancement of core-shell nanoparticles compared to pure Ag nanoparticles was greater than one order of magnitude due to the presence of the pinholes on the surface considered as "hot spots". The performance of Ag core-Au shell nanoparticles was later confirmed by detecting the certain biomolecules at nanomolar concentration range [26]. The metallic core-shell nanoparticles were used not only for DNA sensing based on colorimetric detection [27,28] but also the imaging of the cancer biomarker in live cells by their modification with a Raman reporter molecule and antibody [29].

The USA patent application No.US2004038255 known in the art discloses composite core/shell nanoparticles and a two-step method for preparation thereof. The invention also relates to methods of detection of biomolecules comprising the biomolecule or specific binding substance-core/shell nanoparticle conjugates.

The USA patent application No.US2008213377 known in the art discloses providing systems, methods, and compositions for targeted delivery of nanoparticles and/or agents to tissues, cells, and/or subcellular locales. In general, compositions comprise a nanoparticle (e.g. quantum dot, polymeric particle, etc.), at least one modulating entity (such as a targeting moiety, transfection reagent, protective entity, etc.), and at least one agent to be delivered (e.g. therapeutic, prophylactic, and/or diagnostic agent).

The USA patent application No.US2006234248 known in the art discloses metallic nanoclusters capable of providing an enhanced Raman signal from an organic Raman-active molecule incorporated therein are provided. The nanoclusters may be further functionalized, for example, with coatings and layers, such as adsorption layers, metal coatings, silica coatings, probes, and organic layers. The nanoclusters are generally referred to as COINs (composite organic inorganic nanoparticles) and are capable of acting as sensitive reporters for analyte detection. A variety of organic Raman-active compounds and mixtures of compounds can be incorporated into the nanocluster.

The gold nanoparticles (GNPs) are widely used in sensing and biomedical applications. Most of the time, they are modified with reporter molecules along with biological receptor molecules such as oligonucleotides and antibodies. When they are used in a SERS based sensing and imaging, the additional procedures such as silver staining might be necessary [31-32]. Although the increased size of GNPs up to 50 nm may serve to improve the sensitivity in a SERS experiment [33], the best solution for the problem is to construct nanostructures with overlapping surface plasmons known as a "hot-spots" [34-37]. However, this can only be accomplished by bringing nanoparticles into a few nanometers distances to generate nanometer size gaps [38,39]. The preparation of "hot-spots" using several techniques including lithography was demonstrated [40-46]. However, the current technology is far from preparation of such structures in reproducible maner and mass amounts. Therefore, the utility of molecules such as DNA, peptides and proteins could be a new route for tailoring the gaps in nanostructures.

### Summary of the Invention

The objective of the present invention is to enable preparation of core-shell nanoparticles having improved surface plasmon excitation on nanoparticles that can provide higher SERS activity and thus sensitivity.

Another objective of the invention is to enable the use of the prepared nanoparticles in cellular and biomedical imaging based on surface plasmons and efficient light scattering, photothermal therapy and killing microorganisms with heat.

### Detailed Description of the Invention

"Synthesis of oligonucleotide mediated gold core-silver shell nanoparticles" realized to fulfill the objectives of the present invention is illustrated in the accompanying figures, in which,

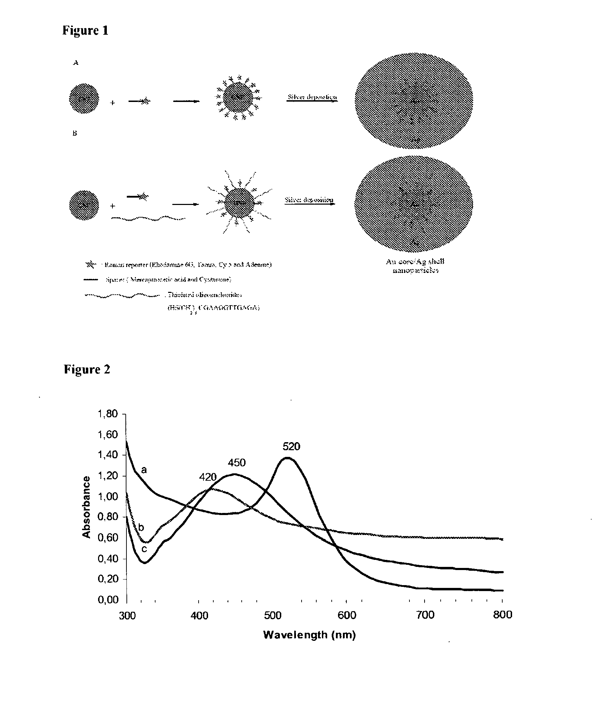

**Figure 1**. Schematic illustration of the preparation of the core-shell nanoparticles with oligonucleotides
**Figure 2**. Absorption spectra of the gold colloidal and core-shell suspensions with different shell thickness
**Figure 3**. TEM image of the core-shell nanoparticles (30±7nm)
**Figure 4**. TEM image of the core-shell nanoparticles (50±10 nm)
**Figure 5**. SERS spectra of the Cy 5 a-with, b-without
**Figure 6**. SERS spectra of the rhodamine 6G a-with, b-without
**Figure 7**. SERS spectra of the adenine a-with, b-without
**Figure 8**. SERS spectra of the TAMRA a-with, b- without an oligonucleotide

The present invention is related to synthesis of oligonucleotide mediated gold core-silver shell nanoparticles and testing their SERS performance for possible sensing, and cellular and biomedical imaging.

The inventive method for synthesis of oligonucleotide mediated gold core-silver shell nanoparticles mainly comprises the following steps;
- synthesizing gold nanoparticles with an average size of 13 nm by means of citrate reduction method,
- activating molecules, which comprise a carboxyl group such as Rhodamine 6G, Rhodamine B, Cy5, Cy3.5 that will chemically attach to the obtained gold nanoparticles, by O-(N-succinimidyl)-N,N,N,N-tetramethyluronium tetrafluoroborate (TSTU), and N,N-Diisopropylethylamine (DIPEA),
- chemically attaching a molecule such as cystamine comprising amino group and thiol group to the activated dye molecules, from the amino group side,
- attaching a molecule such as mercaptoacetic acid comprising a carboxyl group on one end and thiol group on the other, to molecules such as adenine belonging to amine group, from the carboxyl group side,
- attaching thiol group attached molecules and at least 12 base long thiol group attached oligonucleotides to gold nanoparticles via a 13 nm Au-S- bond,
- coating modified nanoparticles with a silver layer by means of citrate or ascorbic acid reduction,
- measuring the SERS spectrum received from the prepared gold core-silver shell nanoparticles.

In this invention, the synthesis and SERS performance of Au core- Ag shell nanoparticles (CSNPs) by embedding chemically attaching the Raman active reporter molecules and thiolated oligonucleotides to gold core and by depositing a thin layer of silver coating on top of it is demonstrated. The two silver reduction approaches were used to coat the derivatized 13±2 nm gold nanoparticles (GNPs); ascorbic acid alone or mixture of ascorbic acid and sodium citrate.

The molecules possessing a carboxyl group such as Rhodamine 6G, TAMRA and mercaptoacetic were first activated using O-(N-succinimidyl)-N,N,N,N-tetramethyluronium tetrafluoroborate (TSTU) and N,N-Diisopropylethylamine (DIPEA) to attach cystamine as a spacer molecule and adenine. The Cy5 dyes recived in activated forms as N-succinimidyl ester (GE Healthcare, Buckinghamshire, UK). The CSNPs possessing oligonucleotides were prepared at two different shell thicknesses, 17±5 nm and 37 ±10 nm, respectively. The UV/vis spectrum of 13±2 nm gold colloidal suspension shows maxima at 520 nm and as the the thickness of silver layer increases the absortion maxima shifts to lower wavelengths to 450 and 420 from 520 nm. The color change during this process can also be observed.

In order to fulfill the objectives of the invention; gold nanoparticles (GNPs) were first synthesized by the citrate reduction of HAuCl₄·3H₂O and this procedure generates an average size of 13 nm GNPs. Briefly, a 50 ml of 38.8 mM sodium citrate solution was added into boiling 500 ml of HAuCl₄·3H₂O (1mM) solution. This solution was boiled for 15 min to complete the reduction of gold salt. The activation of carboxyl groups of rhodamine 6G, TAMRA and mercaptoacetic acid were accomplished by using O-(N-succinimidyl)-N,N,N,N-tetramethyluronium tetrafluoroborate (TSTU), and N,N-Diisopropylethylamine (DIPEA). The Cy 5 N-succinimidyl ester was purchased from GE Healthcare (Buckinghamshire, UK). The spacer molecule was cystamine or a similar molecule possessing thiol group on one end and an mino group on the other ens, between GNPs for the activated dyes such as Rhodamine 6G, TAMRA, Cy 5. The spacter molecule was mercaptoacetic acid for adenine molecule. A 9 ml of acetonitrile was used to dissolve Rhodamine 6G, TAMRA and mercaptoacetic acid (4.65 x 10⁻⁶ mol) and a 8 x 10⁻⁶ mol of TSTU and 29 µmol of 150 µl of DIPEA were added to activate carboxylic groups into the acetonitrile solution. The reaction mixture was kept sterring at room temperature for 24 hours. For already activated dyes such as the Cy 5 N-succinimidyl ester, they were dissolved in 9 ml acetonitrileas they received. In the final step, the cystamine was added into the activated dye solutions and kept sterring at room temperature for 24 hours. For the adenine molecule, it was added into the activated mercaptoacetic acid solution. These procedures generates thiol group possessing Raman active reporter molecules.

In the second step, the thiolated Raman reporter molecules with and without oligonucleoties were prepared. The GNPs were only modified with Raman reporter molecules by mixing a 180 µl of thiolated Raman reporter solution and 13±2 nm gold colloidal suspension to 2 mL of final solution. The GNPs with Raman reporter molecules and oligonucleotides were mixed into a total volume of 2 mL using a 90 µl of thiol modified Raman reporter solution, a 17 µL of (HS(CH₂)₆ CGAAGGTTGAGA) thiol modified oligonucleotide (100µM). The mixtures were kept sterring for 24 hours. All modified gold nanoparticles were centrifuged and washed with distilled water three times at 10000 rpm for 30 min. to remove the unattached Raman reporter molecules and oligonucleotides. The prepared modified GNPs with Raman reporters with/without oligonucleotides were used to prepare core-shell nanoparticles.
Following these steps, the deposition of Ag shell onto modified GNP surface was performed. The Raman reporter modified GNPs with and without oligonucleotides were coated with a silver layer with two different shell thicknesses (Type 1 and 2) using two different approaches.

**Type 1:** The silver ion was reduced with ascorbic acid³ as 17±5 nm thick Ag shell on GNPs modified with only the Raman reporter molecules and Raman reporter molecules with oligonucleotides. Briefly, a 0.1 ml of ascorbic acid (10⁻² M) was added into a 0.9 ml of modified gold nanoparticles, and a 0.8 ml of silver nitrate (10⁻³ M) was slowly added while the reaction mixture was stirring. The red color of solution changed to greenish, indicating the deposition of Ag layer onto gold nanoparticle surfaces. The average diameter of Au-core/Ag shell nanoparticle was 30±7 nm estimated from TEM images.

**Type 2:** The reduction of silver ions were aaccomplished by using a mixture of citrate and ascorbic acid as a 37±10 nm Ag shell on GNPs modified with only the Raman reporters and Raman reporters with oligonucleotides. A 1 ml of the modified gold nanoparticles without oligonucleotides and a 1 ml of sodium citrate (38.8 mM) were added into a 30 ml of water. When this solution was stirring, a 1.2 ml of silver nitrate (10 mM) and a 0.4 ml of ascorbic acid (100 mM) were added to the solution. The color of solution changed from red to yellowish. The average diameter of core-shell nanoparticles synthesized with this was estimated as 50 ±10 nm from TEM images.

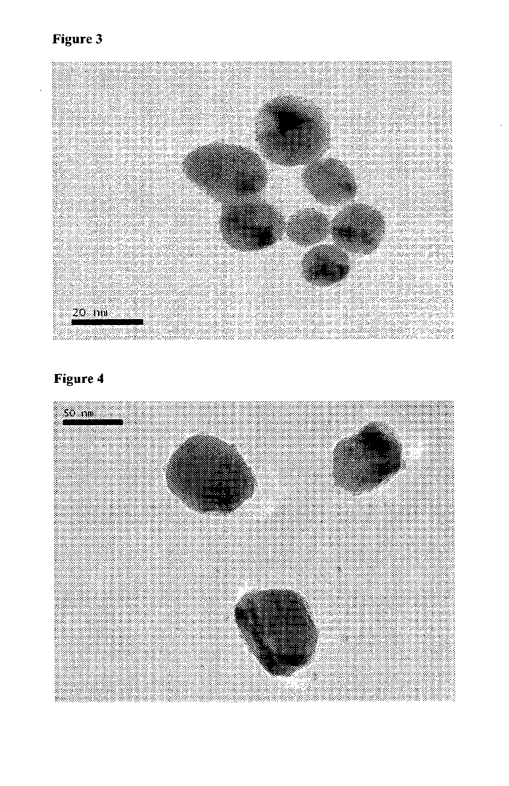

TEM images of the CSNPs prepared with two different shell thicknesses, 17±5nm and 37±10nm, are seen on Figure 3 and 4, respectively. The silver thickness of the type 1 CSNPs were thinner than the type 2 CSNPs. Since the average size of gold core nanoparticles was 13nm, the overall average sizes of CSNPs were 30±7nm and 50±10 as estimated from TEM images (Figure 3 and Figure 4).

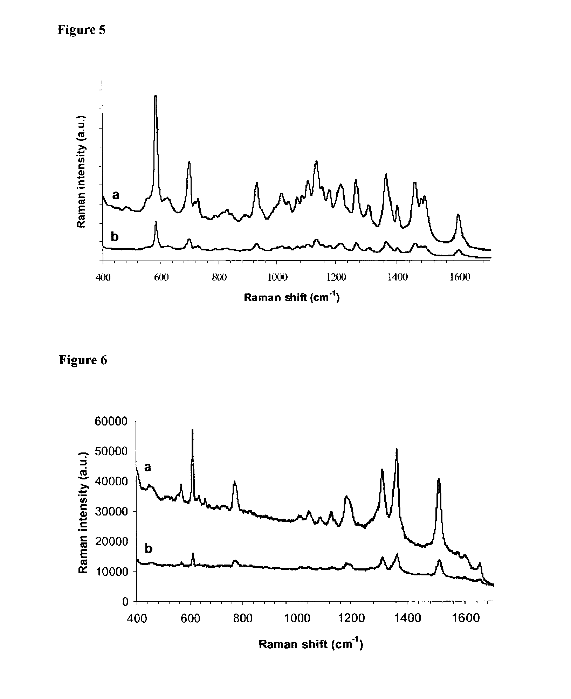

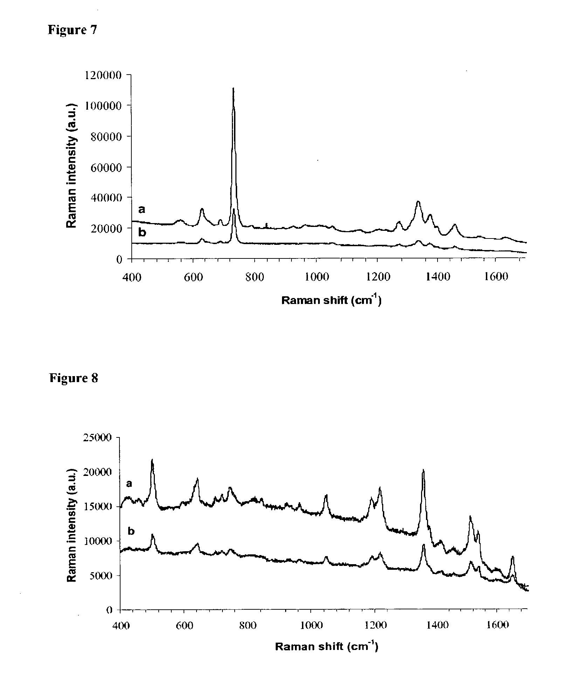

The SERS activity of the CSNPs with 37 ±10 nm shell thickness was not satisfactory. Although, these CSNPs can be used for colorimetric detection, photothermal teraphy and biomedical imaging, thier performance in the SERS experiments were not persued and CSNPs with 17±5 shell thickness were investigated for further SERS experiments. The comparative SERS spectra of of CSNPs prepared with oligonucleotide (a) and these Raman reporter molecules and only (b)Cy 5, Rhodamine 6G, adenine and TAMRA are given in Figure 5-8, respectively.

## Claims

1. Synthesis of oligonucleotide mediated gold core-silver shell nanoparticles **characterized by** the steps of
- synthesizing gold nanoparticles with an average size of 13 nm by means of citrate reduction method,
- activating the carboxyl group of a dye molecule,
- chemically attaching a spacer molecule comprising an amino group on one end and a thiol group on the other end to the activated dye molecule via the amino group in order to prepare a thiol modified dye molecule, and/or
- attaching another spacer molecule comprising an activated carboxyl group on one end and a thiol group on the other end to adenine in order to have a thiol group modified adenine,
- attaching thiol group attached molecules and at least 10 base long thiol group attached oligonucleotides to gold nanoparticles via a 13 nm Au-S-bond,
- coating modified nanoparticles with a silver layer.

2. A gold core-silver shell nanoparticle synthesis according to Claim 1, **characterized in that** the dye molecule is selected among Rhodamine 6G, TAMRA, Rhodamine B, Cy5, Cy3.5.

3. A gold core-silver shell nanoparticle synthesis according to Claim 1 to 2, **characterized in that** the molecules comprising carboxyl group are activated with the O-(N-succinimidyl)-N,N,N,N-tetramethyluronium tetrafluoroborate (TSTU), and N,N-Diisopropylethylamine (DIPEA) molecules.

4. A gold core-silver shell nanoparticle synthesis according to Claim 1 to 3, **characterized in that** the molecule comprising amino group and carboxyl group which chemically attaches to the activated dye molecules from the amino group side is cystamine.

5. A gold core-silver shell nanoparticle synthesis according to Claim 1 to 4, **characterized by** a mercaptoacetic acid comprising a carboxyl group on one end and thiol group on the other, which attaches to the adenine molecule that belongs to amine group, from the carboxyl group side.

6. A gold core-silver shell nanoparticle synthesis according to Claim 1 to 5, **characterized in that** the silver layer coating on the nanoparticles is performed by the citrate or ascorbic acid reduction.

7. A gold core-silver shell nanoparticle obtainable by the process according to Claim 1 to 6, suitable for for colorimetric detection, photothermal teraphy and biomedical imaging.

## Patentansprüche

1. Synthese von Oligonukleotidhaltigen Nanopartikeln mit einem Goldkern und einer Silberbeschichtung **gekennzeichnet durch** die Schritte;
Synthetizieren von Gold-Nanopartikeln mit einer mittleren Größe von 13 nm mittels Citrat-Reduktionsverfahren,
- Aktivieren der Carboxylgruppe eines Farbstoffmoleküls,
- chemisches Hinzufügen eines Spacer-Moleküls umfassend eine Aminogruppe an einem Ende und eine Thiolgruppe am anderen Ende zu den aktivierten Farbstoffmolekülen über eine Aminogruppe um ein **durch** Thiol modifiziertes Farbstoffmolekül bereitzustellen, und/oder,
- Hinzufügen eines anderen Spacer-Moleküls umfassend eine aktivierte Carboxylgruppe an einem Ende und eine Thiolgruppe am anderen Ende zum Adenin, um ein Thiolgruppe modifiziertes Adenin zu erhalten,
- Hinzufügen der mit Thiolgruppen gebundenen Moleküle und der mindestens 10 Basen lange Thiolgruppe zugefügte Oligonukleotide zu den Gold-Nanopartikeln über eine 13 nm Au-S Bindung,
Beschichten der Nanopartikel mit einer Silberschicht.

2. Eine Synthese von Nanopartikeln mit einem Goldkern und einer Silberbeschichtung gemäß Anspruch 1, **dadurch gekennzeichnet, dass** Farbstoffmolekül aus den 6G, TAMRA, Rhodamine B, Cy5, Cy3.5 gewählt wird.

3. Eine Synthese von Nanopartikeln mit einem Goldkern und einer Silberbeschichtung gemäß Ansprüche 1 bis 2, **dadurch gekennzeichnet, dass** die die Carboxylgruppe enthaltende Moleküle mit den O-(N-Succinimidyl) N,N,N,N-Tetramethyluronium Tetrafluoroborat (TSTU) und N,N-Diisopropylethylamin (DIPEA) Molekülen aktiviert sind.

4. Eine Synthese von Nanopartikeln mit einem Goldkern und einer Silberbeschichtung gemäß Ansprüche 1 bis 3, **dadurch gekennzeichnet, dass** das Molekül umfassend die Aminogruppe und Carboxylgruppe, welches an die aktivierten Farbstoffmoleküle von der Seite der Aminogruppen gebunden wird, Cystamin ist.

5. Eine Synthese von Nanopartikeln mit einem Goldkern und einer Silberbeschichtung gemäß Ansprüche 1 bis 4, **gekennzeichnet durch** eine Mercaptoacetische Säure umfassend eine Carboxylgruppe an einem Ende und Thiolgruppe am anderen Ende, wobei sie an Adenin-Molekül gebunden wird, welches der Amingruppe von der Seite der Carboxylgruppe angehört.

6. Eine Synthese von Nanopartikeln mit einem Goldkern und einer Silberbeschichtung gemäß Ansprüche 1 bis 5, **dadurch gekennzeichnet, dass** die Silberschicht, die auf den Nanopartikeln aufgetragen ist, durch Citrat- bzw. Askorbinsäure-Reduzierungsverfahren ausgeführt wurde.

7. Ein Nanopartikel mit einem Goldkern und einer Silberbeschichtung, das durch ein Verfahren gemäß Ansprüche 1 bis 6 erhältlich ist, eignet sich für kolorimetrische Erkennung, fotothermische Behandlung und biomedizinische Abbildung.

## Revendications

1. La synthèse des nanoparticules de noyau en or - enveloppe en argent médiés par oligonucléotide, **caractérisée par** les pas suivants:
- La synthèse des nanoparticules d'or de taille 13 nm en moyenne par voie de la méthode de réduction du citrate,
- L'activation du groupement de carboxyle appartenant à une molécule colorante,
- L'attachement d'une molécule d'espacement consistant en un groupement amino sur l'une extrémité et un groupement thiol à la molécule colorante activée au moyen d'un groupement amino afin de préparer une molécule colorante modifiée par thiol dans une manière chimique, et/ou
- L'attachement d'une autre molécule d'espacement consistant en un actif groupement carboxylé sur l'une extrémité et un groupement thiol sur l'autre extrémité à l'adénine afin d'obtenir un groupement modifiée thiol d'adénine,
- L'attachement des molécules attachées au groupement thiol et des oligonuclétides attachés au groupement thiol long d'au moins 10 bases aux nanoparticules d'or au moyen d'une liaison d'Au-S simultanément,
- Le revêtement des nanoparticules modifiées avec une couche en argent.

2. La synthèse des nanoparticules de noyau en or - enveloppe en argent selon la revendication 1, **caractérisée par** la sélection du groupement carboxyle lié chimiquement aux nanoparticules d'or parmi Rhodamine 6G, TAMRA, Rhodamine B, Cy5, Cy3.5.

3. La synthèse des nanoparticules de noyau en or - enveloppe en argent selon la revendication 1 à 2, **caractérisée par** l'activation des molécules contenant un groupement carboxyle par les molécules O-(N-succinimidyl-N,N,N,N-tetramethyluronium tetrafluoroborate (TSTU) et N,N-Diisoprpoylethylamine (DIPEA).

4. La synthèse des nanoparticules de noyau en or - enveloppe en argent selon la revendication 1 à 3, **caractérisée par le fait que** la molécule contenant un groupement amino lié chimiquement aux molécules colorantes activées du groupement amino et un groupement carboxyle est la cystamine.

5. La synthèse des nanoparticules de noyau en or - enveloppe en argent selon la revendication 1 à 4, **caractérisée par** l'acide mercaptoacétique lié du groupement carboxyle à la molécule d'adénine appartenant au groupement amine, avec un groupement carboxyle à l'une extrémité et un groupement thiol à l'autre.

6. La synthèse des nanoparticules de noyau en or - enveloppe en argent selon la revendication 1 à 5, **caractérisée par** la réalisation du revêtement à couche en argent sur les nanoparticules au moyen de la réduction du citrate ou de l'acide ascorbique.

7. Les nanoparticules de noyau en or - enveloppe en argent pouvant être obtenues par le processus selon la revendication 1 à 6, utilisables pour la détermination calorimétrique, la thérapie photo-thermique, et l'imagerie biomédicale.
